# EUROPEAN PATENT SPECIFICATION

(11) **EP 2 507 212 B1**
(45) Date of publication and mention of the grant of the patent: **16.04.2014**
(21) Application number: 10834318.7
(22) Date of filing: 30.11.2010
(51) Int. Cl.: C07D 211/32

(54) **PROCESS FOR PREPARING METHYL PHENIDATE HYDROCHLORIDE**
VERFAHREN ZUR HERSTELLUNG VON METHYLPHENIDATHYDROCHLORID
PROCÉDÉ DE PRÉPARATION DE CHLORHYDRATE DE PHÉNIDATE DE MÉTHYLE

(30) Priority: 01.12.2009 IN MU27792009
(43) Date of publication of application: 10.10.2012
(73) Proprietor: Harman Finochem Limited, Mumbai 400 098, Maharashtra (IN)
(72) Inventor: JAIN, Kirti, Prakash, Rajasthan (IN); APAR, Shrikrishna, Motiram, Maharashtra (IN); MINHAS, Harpreet, Singh, Maharashtra (IN)
(74) Representative: Isarpatent
(86) International application number: PCT/IN2010/000774
(87) International publication number: WO 2011/067783

(56) References cited:
- IN-A- 2779M UM2 009
- US-A1- 2006 135 777
- US-B1- 6 359 139
- HOWARD M. DEUTSCH ET AL.: 'Synthesis and Pharmacology of Potential Cocaine Antagonists. 2. Structure-Activity Relationship Studies of Aromatic Ring-Substituted Methylphenidate Analogs' J. MED. CHEM. vol. 39, 1996, pages 1201 - 1209
- NNERLY S. PATRICK ET AL.: 'Synthesis and Pharmacology of ydroxylated Metabolites of Methylphenidate' J. MED. CHEM. vol. 24, 1981, pages 1237 - 1240

## Description

### Field of the invention:

The invention relates to a process for the preparation of threo 2-substituted piperidines. In particular, the present invention is directed to a process for the preparation of threo methyl phenidate hydrochloride.

### Background and prior art:

Methylphenidate is the most commonly prescribed psycho stimulant and is indicated in the treatment of Attention deficit hyperactive disorder and also acts as central nervous system stimulant. Originally it was marketed as a mixture of two racemates, 80% (±)-erythro and 20% (±)-threo. Subsequent studies of the racemates indicated that the central stimulant activity was however associated with the threo racemate.

Many synthetic routes are known in the prior art for the preparation of 2- substituted piperidines including methyl phenidate.

U.S Pat. Nos.2507631 and 2957880 discloses multi step synthesis of methyl phenidate (methyl α-piperid-2-yl phenyl acetate) wherein, α-pyrid-2-ylphenyl acetamide (formed initially by reaction of 2-chloro pyridine and acetonitrile followed by hydration in presence of acid) is catalytically hydrogenated to yield α-piperid-2-ylphenyl acetamide. In US2507631 and US2957880, α-piperid-2-yl-phenyl acetamide is separated into threo and erythro diasteromeric racemates through evaporation of solvent; addition of sodium hydroxide to form α-piperid-2-ylphenyl acetamide free base and preferential crystallization of the erythro form by passing gaseous HCl in presence of ethanolic solution. The isolated erythro racemate is then resolved through formation of L-tartarate salt which is further subjected to epimerization to yield desired d-threo diastereomer. Thus according to the procedure disclosed in the said patent, α-piperid-2-ylphenyl acetamide is converted to d-threo methyl α-piperid-2-ylphenyl acetate through hydrolysis and esterification. However, the process is uneconomical in view of the fact that it involves discarding of threo α-piperid-2-ylphenyl acetamide racemate which is isolated following the recrystallization step.

USPat.No.6359139 teaches preparation of a mixture of piperidyl acetamide stereo isomers from /-threo-piperidyl acetamide by reacting with an alkanoic acid. The mixtures of piperidyl acetamide stereo isomers are then contacted with an organic base wherein erythro piperidyl acetamide is converted to threo piperidyl acetamides. The said threo piperidyl acetamides are reacted further with an acid resolving agent thereby precipitating d-threo piperidyl acetamide. The precipitated d-threo piperidyl acetamide is treated with c.H₂SO₄ and methanol to give free base which is further converted to its acid addition salt by bubbling HCl gas.

The another process variant of the said patent involves initial resolution of d, l-threo piperidyl acetamide stereoisomer with an acid resolving agent in an organic solvent thereby forming acid salts of d-threo stereoisomer. The acid salts obtained are further treated with aq. base to precipitate the corresponding piperidyl amide free base which is further reacted with an alcohol in presence of acid to form the corresponding methyl ester. However the overall yield is significantly low and thereby proportional increase in the cost.

EP1607388 describes hydrogenation of α-phenyl-α-pyridyl-2-methylacetate with 5%Pd/C in HClO4/acetic acid to α-phenyl- α-piperidyl-2-methylacetate (methyl phenidate). Methyl phenidate is converted to its hydrochloride salt, wherein the process includes reacting methyl phenidate with dry HCl in IPA at 10-15° C.

EP0948484 teaches preparation of methyl phenidate hydrochloride (98%ee) wherein, to an enriched d-threo methyl phenidate in methanol, HCl gas is bubbled to yield the acid addition salt. J. Med.Chem., 1996, 39, 1201-1209 describes a process for preparing methyl phenidate hydrochloride starting from a threo/erytho mixture of the hydrochloride salt of α-phenyl-α-piperidyl acetamide.

Consequently there remains a need in the art to develop an alternate route of synthesis of methyl phenidate hydrochloride which produces threo methyl phenidate hydrochloride selectively and overcomes with the drawback of prior art processes like multiple step synthesis, use of hazardous metal catalyst and low carbon efficiency.

### Object of the invention:

It is an object of the present invention to provide a method for the preparation of methyl phen idate hydrochloride that overcomes the drawbacks of the processes of the prior arts.

### Summary of the invention:

In line with the object of the invention the process of the current invention discloses a two stage novel method for the preparation of methyl phenidate hydrochloride of Formula I,

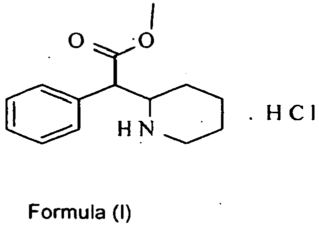

In a preferred aspect, the process for preparation of methyl phenidate hydrochloride (Formula I) comprise the following stages:
Stage I: Acidic hydrolysis of a mixture of α-phenyl- a piperidyl acetamide (Formula II) using 20% aq. solution of Hydrochloric acid , at reflux temperature followed by neutralization with an alkali to precipitate threo-α-phenyl-α-piperidyl-2-acetic acid (Formula III) and; 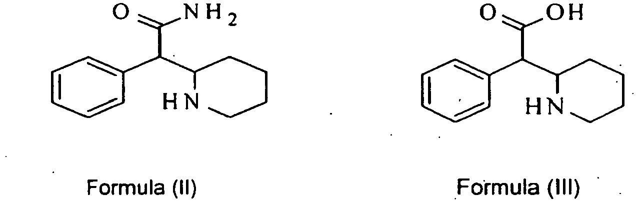
Stage II: Esterification of threo- α-phenyl- α-piperidyl-2-acetic acid (Formula III) using acidic catalyst in methanol followed by treatment with IPA. HCl to precipitate methyl phenidate Hydrochloride.

In an aspect of the present invention, α-phenyl- a piperidyl acetamide (Formula II) used is a mixture of 90% threo isomer and 10% erythro isomer.

### Detailed description:

The present invention provides a two stage process for the preparation of methyl phenidate Hydrochloride Formula I;

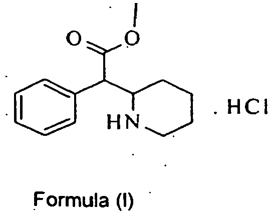

In an embodiment, the two step process for the preparation of Methyl phenidate Hydrochloride consists;
1. Hydrolysis of α-phenyl- α piperidyl acetamide (Formula II) in acidic medium to produce threo - α-phenyl- α piperidyl acetic acid (Formula III)
2. Methyl Ester formation of threo- α-phenyl- α piperidyl acetic acid (Formula III) in presence of catalytic thionyl chloride, methyl ester thus produced is subsequently converted into hydrochloride salt using alcoholic hydrochloric acid solution.

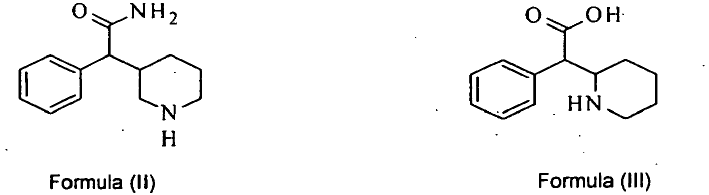

In one of the embodiment of the present invention, mixture of α-phenyl- α piperidyl acetamide (formula II) and 20% aq. solution of hydrochloric acid is heated to reflux, followed by cooling to ambient temperature and dilution with water to get clear solution. This aqueous mass is being washed with dichloromethane for removal of impurities generated during hydrolysis and finally treated with activated carbon. pH of the aq. mass thus obtained should be adjusted to neutral using dilute caustic solution to precipitate threo-α-phenyl-α-piperidyl-2-acetic acid (formula III)

In another embodiment, threo-α-phenyl-α-piperidyl-2-acetic acid (Formula II) is converted to methyl phenidate hydrochloride by treating threo -α-phenyl-α-piperidyl-2-acetic acid (formula II) with methanol in presence of an acid catalyst followed by adding hydrochloric acid solution in IPA.

In yet another embodiment, the acid catalyst used is thionyl chloride. The temperature for the hydrochloride salt formation in stage II is maintained between 0-10°C.

Accordingly, methyl phenidate hydrochloride is prepared wherein, the intermediate threo-α-phenyl-α-piperidyl-2-acetic acid (Formula III) is esterified with methanol in presence of thionyl chloride by maintaining the temperature between 0 - 10°C. The temperature of reaction mass gradually increased to room temp and stirred overnight. After completion of the reaction the solvent was removed under reduced pressure followed by addition of purified water and ethyl acetate. The pH of the reaction mass was adjusted to 9-10 using aq. sodium hydroxide solution and extracted again with ethyl acetate. The solvent layers were combined and evaporated to dryness. The residue thus obtained was diluted with isopropyl alcohol and acidified using alcoholic hydrochloric acid solution. The product thus precipitated was washed with 2-3 times with isopropyl alcohol, the wet cake air dried to get methyl phenidate hydrochloride (Formula I).

Further details of the process of the present invention will be apparent from the examples presented below. The examples presented are purely illustrative and are not limited to the particular embodiments illustrated herein but include the permutations, which are obvious as set forth in the description.

### Example 1: Preparation of threo- α-phenyl-α-piperidyl-2-acetic acid (Formula III)

The reactor was charged with a mixture of α-phenyl-α-piperidyl acetamide (100 g) with 20 % aqueous solution of hydrochloric acid (500 ml) and was heated to reflux for 2-6 hr till completion of the reaction. The reaction mixture was cooled to ambient temperature followed by dilution with water (950 ml) to get clear solution. To the clear solution was added with constant stirring dichloromethane (200 ml) for 30 min, and the layers were separated. Aqueous layer thus obtained was treated through activated carbon, stirred for 30 min and filtered. The pH of the filtrate was adjusted to 6.0-7.0 using aqueous sodium hydroxide solution to yield 88.2 g (88.6 %) of threo-α-phenyl-α-piperidyl-2-acetic acid (Formula III).
(Chromatographic purity by HPLC >99.5%)
(Isomeric purity Threo-99.9% Erythro 0.10%)

### Example II: Preparation of Methyl Phenidate Hydrochloride

To a solution of threo-α-phenyl-α-piperidyl-2-acetic acid (75 g) (From examplel) and methanol (750 ml) was added thionyl chloride (83 g) maintaining the temperature below 10°C. The reaction mass was kept under stirring over night at room temperature. Excess methanol was then distilled off under reduced pressure and the reaction mass was further cooled to 10°C. Purified water (900ml) was then charged maintaining the temperature between 0-10°C followed by addition of ethyl acetate (375ml) under constant stirring. pH of the mixture was made alkaline using dilute caustic solution, with stirring followed by separation of the layers. The separated aqueous layer was once again extracted with ethyl acetate (225ml). The combined ethyl acetate layers were then washed with water and the solvent was distilled off completely. To the residue was charged Isopropyl alcohol (225 ml) and cooled to 0-5°C .The reaction mass was then acidified by adding hydrochloric acid (25% solution in isopropanol) at 0-10°C. The temperature was raised to 25°C, stirred at this temperature for 2-4 hrs, cooled the reaction mass again to 0-10°C and stirred for 2 hrs. The precipitated solid was filtered off, washed with chilled isopropyl alcohol and air dried to get 82.2g (89%) of methyl phenidate hydrochloride.
(Chromatographic purity by HPLC > 99.5%)

## Claims

1. A process for the preparation of methyl phenidate hydrochloride (Formula I),. comprising the steps of;
(a) Hydrolyzing α-phenyl-α-piperidyl acetamide (Formula II) in presence of acid at reflux temperature, followed by treating with an alkali solution to yield threo- α-phenyl-α-piperidyl -2-acetic acid (Formula III) and
(b) Converting threo-α-phenyl-α-piperidyl-2-acetic acid to methyl phenidate hydrochloride by reacting with methanol in presence of acid catalyst followed by treatment with HCl in isopropyl alcohol.

2. A process according to claim 1, wherein the acid hydrolysis of compound of Formula II is carried out using 20 % aq. solution of HCl by refluxing the reaction mass for 2-6 hrs.

3. A process according to claim 1, wherein the reaction mass after acid hydrolysis is washed with dichloromethane to remove the impurities generated during the hydrolysis to obtain high purity (>99.5%) threo -α-phenyl-α-piperidyl-2-acetic acid (Formula III).

4. A process according to claim 1, wherein the compound of Formula II is heated to a temperature 90-110°C.

5. A process according to claim 1, wherein α-phenyl-α-piperidyl acetamide (Formula II) is a mixture of 90 % Threo isomer and 10 % Erythro isomer.

6. A process according to claim 1, wherein the catalyst used for conversion of threo -α-phenyl-α-piperidyl -2-acetic acid to its methyl ester is thionyl chloride.

## Patentansprüche

1. Verfahren zur Herstellung von Methylphenidathydrochlorid (Formel I), das folgende Schritte umfasst:
(a) Hydrolysieren von α-Phenyl-α-piperidylacetamid (Formel II) in Gegenwart von Säure bei Rückflusstemperatur, gefolgt von einer Behandlung mit einer Alkalilösung, um Threo-α-phenyl-α-piperidyl-2-Essigsäure (Formel III) zu erhalten, und
(b) Umwandeln von Threo-α-phenyl-α-piperidyl-2-Essigsäure zu Methylphenidathydrochlorid durch Reagieren mit Methanol in Gegenwart eines Säurekatalysators, gefolgt von einer Behandlung mit HCl in Isopropylalkohol.

2. Verfahren nach Anspruch 1, bei dem die Säurehydrolyse der Verbindung von Formel II unter Verwendung von 20 %-iger wässriger Lösung von HCl durch Kochen der Reaktionsmasse am Rückfluss für 2-6 Stunden ausgeführt wird.

3. Verfahren nach Anspruch 1, bei dem die Reaktionsmasse nach der Säurehydrolyse mit Dichlormethan gewaschen wird, um die Verunreinigungen zu entfernen, die während der Hydrolyse entstanden sind, um hoch-reine (> 99,5 %) Threo-α-phenyl-α-piperidyl-2-Essigsäure (Formel III) zu erhalten.

4. Verfahren nach Anspruch 1, bei dem die Verbindung von Formel II auf eine Temperatur 90-110 °C erwärmt wird.

5. Verfahren nach Anspruch 1, bei dem α-Phenyl-α-piperidylacetamid (Formel II) ein Gemisch aus 90 % Threoisomer und 10 % Erythroisomer ist.

6. Verfahren nach Anspruch 1, bei dem der Katalysator, der für die Umwandlung von Threo-α-phenyl-α-piperidyl-2-Essigsäure zu ihrem Methylester verwendet wird, Thionylchlorid ist.

## Revendications

1. Procédé de préparation du chlorhydrate de phénidate de méthyle (formule I), comprenant les étapes suivantes :
(a) l'hydrolyse de l'α-phényl-α-pipéridyl-acétamide (formule II) en présence d'un acide à une température de reflux, puis la mise en oeuvre d'un traitement avec une solution alcaline pour obtenir l'acide thréo-α-phényl-α-pipéridyl-2-acétique (formule III) et
(b) la conversion de l'acide thréo-α-phényl-α-pipéridyl-2-acétique en chlorhydrate de phénidate de méthyle par la mise en oeuvre d'une réaction avec du méthanol en présence d'un catalyseur acide, puis d'un traitement avec du HCl dans de l'alcool isopropylique.

2. Procédé selon la revendication 1, dans lequel l'hydrolyse acide du composé de formule II est réalisée en utilisant une solution aqueuse de HCl à 20% en laissant à reflux la masse réactionnelle pendant 2 à 6 heures.

3. Procédé selon la revendication 1, dans lequel la masse réactionnelle après l'hydrolyse acide est lavée avec du dichlorométhane pour éliminer les impuretés générées lors de l'hydrolyse, afin d'obtenir l'acide thréo-α-phényl-α-pipéridyl-2-acétique (formule III) sous une forme très pure (> 99,5%).

4. Procédé selon la revendication 1, dans lequel le composé de formule II est chauffé à une température de 90°C à 110°C.

5. Procédé selon la revendication 1, dans lequel l'α-phényl-α-pipéridyl-acétamide (formule II) est un mélange contenant 90% d'isomère thréo et 10% d'isomère érythro.

6. Procédé selon la revendication 1, dans lequel le catalyseur utilisé pour la conversion de l'acide thréo-α-phényl-α-pipéridyl-2-acétique en son ester de méthyle est le chlorure de thionyle.
